(19) Europäisches Patentamt
European Patent Office
Office européen des brevets

(11) Publication number: **0 311 387 B1**

(12) # EUROPEAN PATENT SPECIFICATION

(45) Date of publication of patent specification: **11.08.93**

(51) Int. Cl.5: **C08G 73/02**, C08G 73/12

(21) Application number: **88309298.3**

(22) Date of filing: **06.10.88**

(54) **Aromatic amine resins, their production process and thermosetting resin compositions making use of the same.**

(30) Priority: **08.10.87 JP 252517/87**
**10.11.87 JP 282048/87**
**09.12.87 JP 309426/87**

(43) Date of publication of application:
**12.04.89 Bulletin 89/15**

(45) Publication of the grant of the patent:
**11.08.93 Bulletin 93/32**

(84) Designated Contracting States:
**CH DE FR GB IT LI NL**

(73) Proprietor: **MITSUI TOATSU CHEMICALS, Inc.**
**2-5 Kasumigaseki 3-chome**
**Chiyoda-Ku Tokyo 100(JP)**

(72) Inventor: **Yamaguchi, Keizaburo**
**600-1-533, Kamisakunobe Takatsu-ku**
**Kawasaki-shi Kanagawa-ken(JP)**
Inventor: **Tanabe, Yoshimitsu**
**2070 Iijimacho Sakae-ku**
**Yokohama-shi Kanagawa-ken(JP)**
Inventor: **Urakami, Tatsuhiro**
**2212 Senmarudai Danchi 350, Araicho**
**Hodogaya-ku**
**Yokohama-shi Kanagawa-ken(JP)**
Inventor: **Yamaguchi, Akihiro**
**1-13-24, Zaimokuza Kamakura-shi**
**Kanagawa-ken(JP)**
Inventor: **Yamaya, Norimasa**
**2882, Iijimacho Sakae-ku**
**Yokohama-shi Kanagawa-ken(JP)**
Inventor: **Ohta, Masahiro**
**1541 Yabecho Totsuka-ku**
**Yokohama-shi Kanagawa-ken(JP)**

(74) Representative: **West, Alan Harry et al**
**R.G.C. Jenkins & Co. 26 Caxton Street**
**London SW1H 0RJ (GB)**

EP 0 311 387 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel aromatic amine resins, their production, and thermosetting resin compositions making use of them and having excellent mechanical strength without impairment of heat resistance.

Aromatic amine resins have been known for many years as condensation products of aromatic amines and formaldehyde. For example, aniline-formaldehyde resin represented by the following general formula (f):

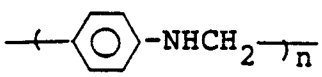

(f)

has been produced [K. Frey, Herbetica Chemie Acta, 18, 481 (1935)].

It is difficult to introduce maleimide or isocyanate groups into conventional aromatic amine resins composed of a secondary amine and represented by the general formula (f), such as aniline-formaldehyde resin. Secondary amines are hence not suitable as raw materials for maleimide resins or isocyanate resins. Conventional aromatic amine resins have therefore been used widely as curing agents. Today, increasingly sophisticated applications and more rigorous expectations of their performance and consistency of performance are placing demands on conventional aromatic amine resins used as curing agents for matrix resins of heat-resistant composite materials, high-temperature-resistant bonding agents and the like. These demands can no longer always be met adequately by those resins.

Heat-resistant composite materials, high-temperature-resistant bonding agents, etc. are required to withstand instantaneous impact such as stress concentration as an external stress. Ideally, the ability of undergoing elastic deformation like rubber is therefore an important property. As a criterion for the judgement of such elastic deformation, the elongation at break of each matrix resin is particularly important. The greater the elongation of the matrix resin, the greater the compensation for drawbacks associated with the use of reinforcing materials such as glass fibers or carbon fibers required for composite materials. In other words, use of a matrix resin of a greater elongation leads to an improvement in the overall strength of a composite material.

For these matrix resins and the like, long-term storage stability is also important in addition to heat resistance and dimensional stability. They are also required to undergo less deterioration by light or oxygen in the air. This oxidation resistance is primarily attributable to the structure of each resin. In addition to the failure in satisfying the above-mentioned demand for mechanical strength, conventional aromatic amine resins involve various drawbacks which are attributed to their structural defects.

The above-described aniline-formaldehyde resin is converted into a crosslinked structure if the molar ratio of formaldehyde is increased upon condensation with a view toward increasing the degree of condensation and improving its mechanical properties and the like. However, it is only possible to increase its molecular weight to 600 or so in general [Noda, et al., Nippon Kogyo Kagaku Zasshi, 55, 484-487 (1952)].

The present inventors have already found novel aromatic amine resins which have improved these deficiencies, on which an application for patent was filed on September 17, 1987 in Japan (Japanese Patent Application No. 230987/1987). Since these resins are composed of a secondary amine, it is difficult to achieve their isocyanation or maleimidation. They are accompanied by further problems such that when used as curing agent, they must be employed in a relatively large amount and their curing speeds are relatively slow.

On the other hand, thermosetting resins having an imide structure have already found wide-spread industrial utility owing to their excellent properties such as electrical insulating properties, heat resistance and dimensional stability of moldings.

Thermosetting resins which are obtained by separately subjecting aromatic bismaleimides to heat polymerization are however accompanied by such drawbacks as extremely brittleness and poor flexibility, although they do have superb heat resistance. As a method for improving such properties, attempts have been made to use a thermosetting resin composition of an aromatic bismaleimide and an aromatic diamine. For example, a polyaminobismaleimide resin ("Kerimid", trade mark; product of Rhone-Poulenc S.A.) composed of N,N'-4,4'-diphenylmethanebismaleimide and 4,4'-diaminodiphenylmethane has been utilised. It is used widely in impregnating varnishes, laminated boards, moldings, etc. (Japanese Patent Publication No. 23250/1971).

However, these thermosetting resin compositions are not sufficient in heat resistance and are not satisfactory either in impact resistance or flexibility.

The present invention seeks to provide not only an aromatic amine resin which when used as a curing agent, provides a cured resin having excellent heat resistance, mechanical strength, dimensional stability, and light and oxygen stability and which is also useful as a starting material for isocyanate resins, maleimide resins and the like which are superb in such properties but also a thermosetting resin composition having excellent mechanical strength and heat resistance.

The invention seeks to provide also a process for producing the aromatic amine resin, which is an improvement over the known process and features greater economy.

In accordance with the invention there is provided an aromatic amine resin formed of a mixture of aromatic amines of the general formula (a):

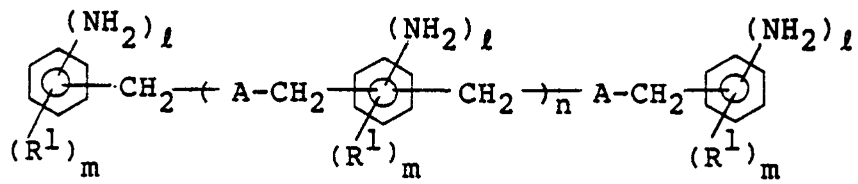

(a)

in which A is phenylene, alkyl-substituted phenylene, diphenylene, diphenyl ether or naphthylene, $R^1$ is halogen or hydroxyl, $C_{1-4}$ alkoxy or $C_{1-5}$ alkyl, $\ell$ is 1 or 2, $m$ is O or an integer from 1 to 3 and when $m$ is 2 or 3, then the $R^1$s may be the same or different and two $R^1$s may together form a 5- or 6-membered alicyclic moiety which may optionally contain one or more side chains, and $n$ has a value from 0 to 300, preferably from 0 to 4.

In accordance with the invention there is provided also a first process for the production of such an aromatic amine resin, which comprises reacting together in the presence of an acid catalyst an aromatic amine of the general formula (b):

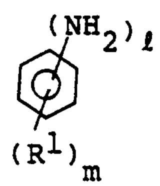

(b)

in which $R^1$, $\ell$ and $m$ have the meanings specified above, and an aralkyl alcohol derivative of the general formula (c):

$R^2OCH_2\text{-}A\text{-}CH_2OR^2$ (c)

in which A has the meaning specified above and $R^2$ is hydrogen, acyl or $C_{1-4}$ alkyl, and wherein:

(i) the compounds (b) and (c) are in a molar ratio of 1 to 15;

(ii) the amount of acid catalyst is 10 to 100 mole % of the aromatic amine;

(iii) the temperature is 130 to 240 °C; and

(iv) the reaction time is 10 to 40 hours.

In accordance with the invention there is provided further a second process for the production of such an aromatic amine resin, which comprises reacting together an aromatic amine of the general formula (b):

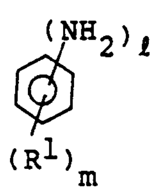

(b)

in which $R^1$, $\ell$ and $m$ have the meanings specified above, and a bishalomethyl derivative of the general formula (d):

$X-CH_2-A-CH_2-X$ (d)

in which A has the meaning specified above and X is halogen, and wherein

(i) the compounds (b) and (c) are in a molar ratio of 1 to 15;
(ii) the temperature is 0 to 240 °C, and
(iii) the reaction time is 6 to 50 hours.

In accordance with the invention there is provided additionally a modification of the second process for the production of such an aromatic amine resin, which comprises reacting together in a two stage process an aromatic amine of the following formula (b):

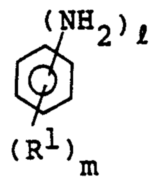

(b)

in which $R^1$, $\ell$ and $m$ have the meanings specified above, and a bishalomethyl derivative of the general formula (d):

$X-CH_2-A-CH_2-X$ (d)

in which A has the meaning specified above and X is halogen, and wherein:

(i) in the first stage, the temperature is 0 to 130 °C and the reaction time is 1 to 10 hours, and
(ii) in the second stage, the temperature is 130 to 240 °C and the reaction time is 5 to 40 hours.

FIG. 1 diagrammatically shows results of an IR analysis of an aromatic amine resin obtained in Example 1.

The aromatic amine resins according to this invention can be used in a wide variety of fields, for example as raw materials for epoxy resins or curing agents for other epoxy compounds, raw materials for maleimide resins or curing agents for other maleimide compounds, raw materials for isocyanate resins or curing agents for other isocyanate compounds, chelate resins, ion-exchange resins, molding materials, insulating paints, bonding agents, rubber modifiers, additives for various resins, deacidification agent, raw materials for polyimides, polyamides and polyamideimides.

Since the aromatic amine resins according to this invention are composed of a primary amine, it is easy to achieve their isocyanation, maleimidation or epoxidation.

Further, resins having high performance can be obtained from the use of the aromatic amine resins of this invention as curing agents for other resins (for example, isocyanate compounds, epoxy compounds, and bismaleimide compounds).

When the resins according to this invention are used as curing agents for bismaleimide compounds, the resultant cured resins are excellent in mechanical strength and dimensional stability and also superb in heat resistance and light and air oxygen stability. Their curing speeds are also high. As one specific example, use of any one of the resins according to this invention as a curing agent for bismaleimide derived from methylenedianiline can provide a cured resin whose flexural strength, flexural modulus, pyrolysis starting temperature in air, coefficient of expansion and water absorption rate are superior to those of "Kerimid 1050" (trade name; molding grade; product of Rhone-Poulanc S.A.) obtained by using methylene-dianiline as a curing agent. Their glass transition temperatures and heat distortion temperatures are substantially comparable.

In addition, the resins according to this invention are soluble in low boiling point solvents (dioxane, methylene chloride, etc.).

A prepreg has heretofore been prepared by dissolving a prepolymer such as "Kerimid" in a high boiling point aprotic polar solvent (N-methylpyrrolidone or the like) and then impregnating glass cloth or carbon cloth with the solution. In contrast to such a conventional method, use of any one of the resins according to this invention as a prepolymer facilitates the evaporation and removal of the solvent to provide an excellent prepreg because the resin can be dissolved in a low boiling point solvent.

The resins according to this invention are faster in curing speed than the resins disclosed previously by the present inventors in Japanese Patent Application No. 230987/1987, so that they are preferred particularly for use as sealing resins for semiconductors.

The resins according to this invention can each be produced by subjecting the aromatic amine compound represented by the general formula (b) and the aralkyl alcohol derivative represented by the general formula (c) to a co-condensation reaction. If aniline and the aralkyl alcohol derivative for example are caused to undergo co-condensation, a resin containing a secondary amine such as that having a structure of the following formula (g):

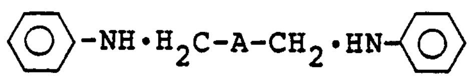

(g)

is formed. It is however only necessary to convert the secondary amine resin into a primary amine resin of this invention by a rearrangement reaction. This rearrangement reaction can be conducted, for example, (a) by increasing the amount of the catalyst, (b) by increasing the reaction temperature or (c) by increasing the reaction time compared to the reaction conditions under which the secondary amine is formed. In particular, it is effective to increase the amount of the catalyst.

As already mentioned above, it has been only possible to increase the molecular weight of the conventionally-known aniline-formaldehyde resin to 600 or so. In contrast, the aromatic amine resins according to this invention may each be chosen as desired from a low molecular resin composed principally of the aromatic amine compound of the general formula (a) in which n is 0 to a high molecular resin composed of the aromatic amine compound principally of the general formula (a) in which n is about 300. As an advantage of the first process of this invention, may be mentioned that aromatic amine resins in various forms, ranging from a resin in a liquid form at room temperature to a high softening point resin in a resinous form, can be produced depending on the end use.

Resins according to this invention, ranging from liquid resins to those having a low softening point, can each be obtained provided that the molar ratio of the aromatic amine to the aralkyl alcohol derivative is increased. The liquid-low softening point resins thus obtained are excellent in workability upon melt blending, impregnation, coating and the like and are hence usable primarily in fields such as bonding agents, paints, and additives for urethane and other resins.

High softening point resins according to this invention can each be obtained provided that the molar ratio of the aralkyl alcohol derivative to the aromatic amine is set in the vicinity of the stoichiometric ratio upon their condensation. The high softening point resins thus obtained are useful in fields such as molding materials, ion-exchange resins and laminating resins.

The molecular weight range of the aromatic amine resins obtained as described above in accordance with the first process of this invention ranges from about 300 to about 60,000. The softening point range of the resins are from a liquid state at room temperature to about 250 °C (as ring and ball softening points measured in accordance with JIS-K-2548).

One specific example of the first process according to this invention will now be described in detail.

First of all, the aromatic amine compound represented by the general formula (b) is added in an amount of 1-15 moles, preferably, 1.1-10 moles per mole to the aralkyl alcohol derivative represented by the general formula (c). They are heated as such in the presence of an acid catalyst and are reacted at a temperature to be described subsequently. Water, alcohol, organic acid and/or the like, which are formed as the reaction proceeds, are trapped outside the system. Volatiles which remain at trace levels within the system may be purged with nitrogen if necessary. When it is desired to obtain a relatively low molecular resin composed principally of the aromatic amine compound of the general formula (a) in which n is 0, it appears to be desirable to make the above-mentioned molar ratio greater. A greater molar ratio however results in more unreacted amine, so that more time and labor are required for its removal after the reaction. The molar ratio of 10 was compared to the molar ratio of 15 while maintaining the other reaction conditions constant. Since the proportion of the aromatic amine compound, in which n was 0, in the resin as a reaction product did not increase very markedly even when the molar ratio was increased to 15, the preferable upper limit of the molar ratio is 10.

Illustrative examples of A in the aralkyl alcohol derivative, which is useful in the practice of this invention and is represented by the general formula (c), include phenylene groups

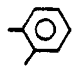, 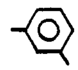 and 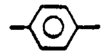;

alkyl-substituted phenylene groups

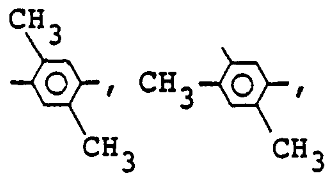 and

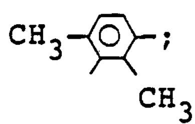

diphenylene group

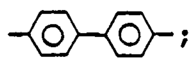

diphenyl ether group

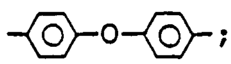

and naphthylene group

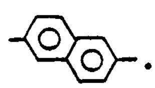

$R^2$ is a hydrogen atom or an acyl or alkyl group. Regarding the acyl or alkyl group represented by $R^2$, the reaction rate is high with a carbon number of 4 or smaller. When the carbon number is 4, namely, $R^2$ is a butyl group, tert-butyl group tends to give a low reaction velocity. As illustrative examples of aralkyl alcohol derivatives useful in the practice of this invention, may be mentioned α,α'-dihydroxy-o-xylene, α,α'-dihydroxy-m-xylene, α,α'-dihydroxy-p-xylene, α,α'-diacetoxy-o-xylene, α,α'-diacetoxy-m-xylene, α,α'-diacetoxy-p-xylene, α,α'-dipropionoxy-p-xylene, α,α'-di-n-butyloxy-p-xylene, α,α'-dimethoxy-o-xylene, α,α'-dimethoxy-m-xylene, α,α'-dimethoxy-p-xylene, α,α'-diethoxy-o-xylene, α,α'-diethoxy-m-xylene, α,α'-diethoxy-p-xylene, α,α'-diisopropoxy-o-xylene, α,α'-diisopropoxy-m-xylene, α,α'-diisopropoxy-p-xylene, α,α'-di-n-propoxy-p-xylene, α,α'-di-n-butoxy-m-xylene, α,α'-di-n-butoxy-p-xylene, α,α'-di-sec-butoxy-p-xylene, α,α'-diisobutoxy-p-xylene, 4,4'-dihydroxymethyldiphenyl ether, 4,4'-dihydroxymethyldiphenyl, 2,6-dihydroxymethylnaphthalene, 4,4'-diacetoxymethyldiphenyl ether, 4,4'-diacetoxymethyldiphenyl, 2,6-diacetoxymethylnaphthalene, 4,4'-methoxymethyldiphenyl ether, 4,4'-methoxymethyldiphenyl, 4,4'-diethoxymethyldiphenyl ether, 4,4'-diisopropoxymethyldiphenyl, 4,4'-diisobutoxymethyldiphenyl ether, α,α'-dimethoxy-2-methyl-p-xylene, α,α'-dimethoxy-3-methyl-m-xylene, α,α'-dihydroxy-2,5-dimethyl-p-xylene, α,α'-dimethoxy-2,5-dimethyl-p-xylene, α,α'-dimethoxy-2,4-dimethyl-1,3-xylene and α,α'-dimethoxy-2,4-dimethyl-1,5-xylene. α,α'-Dimethoxy-p-xylene is the most suitable compound.

In the aromatic amine compound which is represented by the general formula (b) and is useful in the practice of this invention, $R^1$ is a halogen atom or a hydroxyl, $C_{1-4}$ alkoxy or $C_{1-5}$ alkyl group. The aromatic amine compound may contain 0-3 $R^1$s. When 2-3 $R^1$s are contained, they may be either the same or different. Two of the $R^1$s may join together to form a 5-membered or 6-membered alicyclic group which may contain one or more side chains. The aromatic amine compound contains one or two amino groups As specific examples of the aromatic amine compound, may be mentioned aniline, o-toluidine, m-toluidine, p-toluidine, o-ethylaniline, m-ethylaniline, p-ethylaniline, o-isopropylaniline, m-isopropylaniline, p-isopropylaniline, o-n-propylaniline, o-tert-butylaniline, p-tert-butylaniline, o-n-butylaniline, p-sec-butylaniline, 2,3-xylidine, 2,4-xylidine, 2,6-xylidine, 3,4-xylidine, 3,5-xylidine, 2-methyl-3-ethylaniline, 2-methyl-4-isopropylaniline, 2,6-diethylaniline, 2-ethyl-5-tert-butylaniline, 2,4-diisopropylaniline, 2,4,6-trimethylaniline, 4-chloroaniline, 4-bromoaniline, 4-fluoroaniline, 3-chloroaniline, 3-bromoaniline, 3,4-dichloroaniline, 3-chloro-o-toluidine, 3-chloro-p-toluidine, 2,6-dimethyl-4-chloroaniline, o-aminophenol, m-aminophenol, p-aminophenol, 2-amino-4-cresol, 4-amino-2-tert-butylphenol, 2,6-dimethyl-4-aminophenol, 2,6-dichloro-4-aminophenol, 2-amino-1,3-resorcin, 4-amino-1,3-resorcin, 2-aminohydroquinone, 2-methoxyaniline, 3-methoxyaniline, 4-methoxyaniline, 2-isopropoxyaniline, 2,4-dimethoxyaniline, o-phenylenediamine, m-phenylenediamine, p-phenylenediamine, 2,4-diaminotoluene, 2,6-diaminotoluene, 2,4-diaminoethylbenzene, 2,6-diaminoethylbenzene, 2,4-diaminoisopropylbenzene, 2,4-diamino-tert-butylbenzene, 2,6-diamino-tert-butylbenzene, 2,4-diamino-1,3-dimethylbenzene, 1,1-dimethyl-4-aminoindane and 1,1-dimethyl-4,6-diaminoindane. Preferred compounds are aniline, toluidines, xylidines, aminophenol and diamines, with aniline being particularly preferred.

As the acid catalyst, it is possible to use either singly or in combination an inorganic or organic acid, especially a mineral acid, e.g., hydrochloric acid, phosphoric acid, sulfuric acid or nitric acid, a Friedel-Crafts catalyst such as zinc chloride, stannic chloride, aluminum chloride or ferric chloride, an organic sulfonic acid such as methanesulfonic acid or p-toluenesulfonic acid, or a super strong acid such as trifluoromethanesulfonic acid or "Nafion H" (trade name; product of E.I. du Pont de Nemours & Co., Inc.). Besides, a solid acid catalyst such as an activated clay or zeolite, or a heteropolyacid may also be used. Preferred industrially is hydrochloric acid for its economical price. The catalyst may be used in an amount of 10 mole % or more, preferably, 20-100 mole % based on the aromatic amine compound. Any amounts less than the lower limit result in a slow reaction rate and difficulty in achieving complete conversion into primary amine compounds. Amounts greater than the upper limit do not give adverse effects to the reaction but are not economical.

The reaction temperature may preferably be 130°C or higher. The reaction becomes extremely slow if the temperature is lower than 130°C. A temperature range of about 170-240°C is desirable in order to shorten the reaction temperature as much as possible. The reaction time may range from 10 hours to 40 hours. As a resin having greater values of $\underline{n}$ in the general formula (a) is produced, the reaction time becomes shorter.

Incidentally, an inert solvent may be used in the reaction of the first production process according to this invention. However, the reaction is usually carried out without solvent. After completion of the reaction, the acid employed as a catalyst is neutralized, for example, with a dilute aqueous alkaline solution such as an aqueous solution of caustic soda, an aqueous solution of potassium hydroxide or aqueous ammonia and is then separated.

When unreacted aromatic amine compound remains, it is distilled out in vacuum or by steam distillation.

According to the second process of this invention, the aromatic amine compound represented by the general formula (b) and the bishalomethyl derivative represented by the general formula (d) can still be reacted in two stages in the absence of a catalyst. In this case, a resin containing a secondary amine is formed in the first-stage reaction. It is however only necessary to convert the secondary amine into a primary amine resin by a rearrangement (second-stage reaction). This rearrangement is conducted using a hydrogen halide, which occurs upon formation of the primary amine resin, as a catalyst. However, in order to accelerate this rearrangement, the reaction is conducted in the same manner as in the first process of this invention, for example (a) by increasing the amount of catalyst, (b) by increasing the reaction temperature or (c) by prolonging the reaction time, compared to the reaction conditions under which a secondary amine resin is formed. It is particularly effective to increase the amount of the catalyst.

Like the first process, the second process of this invention has also made it possible to freely produce aromatic amine resins, which range from a low molecular resin composed principally of the aromatic amine compound of the general formula (a) in which $\underline{n}$ is 0 to a high molecular resin composed principally of the aromatic amine compound of the general formula (a) in which $\underline{n}$ is about 300, by changing the molar ratio of the aromatic amine compound to the bishalomethyl derivative in the condensation. As an advantage of the second process of this invention, it is also mentioned that aromatic amine resins in various forms ranging from those in a liquid form at room temperature to those having a high softening point and resinous appearance can be produced in accordance with intended end use. Thus, aromatic amine resins ranging from liquid resins to low softening point resins can each be obtained when the molar ratio of the aromatic amine compound to the bishalogenomethyl derivative is increased in the condensation. On the other hand, aromatic amine resins having a high softening point can each be obtained when the molar ratio of the

bishalogenomethyl derivative to the aromatic amine is set near the stoichiometric ratio in the condensation.

The molecular weight range and softening point range of the aromatic amine resins obtained in accordance with the second process of this invention are similar to those of the resins obtained in accordance with the first process.

One specific example of the second process of this invention for the production of an aromatic amine resin will now be described in detail.

First of all, the aromatic amine compound represented by the general formula (b) is added in an amount of 1-15 moles, preferably, 1.1-10 moles per mole to the bishalomethyl derivative represented by the general formula (d). They are heated as such, so that they are reacted at a temperature to be described subsequently. A suitable acid catalyst may be added in advance or during the course of the reaction in order to accelerate the reaction.

In the bishalomethyl derivative which is useful in the practice of the second process and is represented by the general formula (d), A is a phenylene group, an alkyl-substituted phenylene group, a diphenylene group, a diphenyl ether group, a naphthylenyl group or the like, while X is a chlorine, bromine, fluorine or iodine atom. As illustrative examples of bishalogenomethyl derivatives useful in the practice of this invention, may be mentioned α,α'-dichloro-o-xylene, α,α'-dichloro-m-xylene, α,α'-dichloro-p-xylene, α,α'-dibromo-o-xylene, α,α'-dibromo-m-xylene, α,α'-dibromo-p-xylene, α,α'-difluoro-o-xylene, α,α'-difluoro-m-xylene, α,α'-difluoro-p-xylene, α,α'-diiodo-o-xylene, α,α'-diiodo-m-xylene, α,α'-diiodo-p-xylene, 4,4'-bis-(chloromethyl)-diphenyl ether, 4,4'-bis(chloromethyl)diphenyl, 2,6-bis(chloromethyl)naphthalene, 4,4'-bis-(bromoethyl)-diphenyl ether, 4,4'-bis(bromomethyl)diphenyl, 2,6-bis(bromomethyl)naphthalene, 4,4'-bis-(fluoromethyl)-diphenyl ether, 4,4'-bis(fluoromethyl)diphenyl, 4,4'-bis(iodomethyl)diphenyl ether, 4,4'-bis-(iodomethyl)-diphenyl, α,α'-dichloro-2-methyl-p-xylene, α,α'-dichloro-3-methyl-m-xylene, α,α'-dichloro-2,5-dimethyl-p-xylene, α,α'-dibromo-2,5-dimethyl-p-xylene, α,α'-dichloro-2,4-dimethyl-1,3-xylene and α,α'-dichloro-2,4-dimethyl-1,5-xylene. α,α'-Dichloro-p-xylene is preferred.

As $R^1$ in the aromatic amine compound of the general formula (b) employed in this invention, an atom or group similar to that used in the first process may be chosen as desired depending on the final aromatic amine resin desired. One or two amino groups may also be present. Specific examples and the most preferred compound among such specific examples are hence as described above.

In the second process of this invention, an acid catalyst may also be used with a view to accelerating the reaction as mentioned above. As the acid catalyst, any one of acid catalysts usable in the first process can be used. Industrially preferred is hydrochloric acid for its economical price. To the hydrogen halide which is formed in the course of the reaction, the catalyst may be added in an amount of 100 mole % or less based on the aromatic amine compound. Although no problems would arise even if the catalyst is added beyond that level, use of the catalyst in such an excess amount is not economical.

The reaction temperature may range from 0 °C to 240 °C in the entire course of the reaction. However, it may be 0-130 °C, preferably, 20-100 °C in the first-stage reaction and 130-240 °C in the second-stage reaction. In order to shorten the reaction time of the second stage as much as possible, a temperature range of 170-240 °C is desirable. The reaction time may be 1-10 hours in the first stage and 5-40 hours in the second stage. The reaction time also becomes shorter as a resin having greater $\underline{n}$ in the general formula (a) is produced.

Incidentally, an inert solvent may also be used in the reaction of the second process according to this invention. However, the reaction is usually carried out without solvent. After completion of the reaction, the acid formed in the reaction or the acid employed as a catalyst is neutralized, for example with a dilute aqueous alkaline solution such as an aqueous solution of caustic soda, an aqueous solution of potassium hydroxide or aqueous ammonia and is then separated.

When unreacted aromatic amine compound remains, it is distilled out in vacuum or by steam distillation.

The second process of this invention has the advantages that since the bishalomethyl derivative represented by the general formula (d) is used as a starting material, a catalyst is not absolutely essential for the reaction, the aromatic amine resin can be produced at a more economical price, and the process allows less side reactions to occur.

Thermosetting resin compositions according to this invention will now be described.

N,N'-4,4'-diphenylmethanebismaleimide represented by the formula (e) can be prepared with ease by subjecting 4,4'-diaminodiphenylmethane and maleic anhydride to a condensation/dehydration by a method known per se in the art.

A thermosetting resin composition is then obtained from N,N'-4,4'-diphenylmethanebismaleimide represented by the formula (e) and the aromatic amine resin composed of a mixture of aromatic amine compounds represented by the formula (a). Here, the following various methods can each be used.

(1) The bismaleimide and aromatic amine resin are ground and mixed in solid-solid forms or in solid-liquid forms. As an alternative, they are heat-treated into a prepolymer, followed by grinding into pellets or powder. In this case, it is preferred to choose such heating conditions that the resultant mixture is partially cured to the stage of the prepolymer. It is generally suitable to heat them at 70-220 °C for 5-240 minutes with heating at 80-200 °C for 10-180 minutes being preferred.

(2) The bismaleimide and aromatic amine resin are dissolved in an organic solvent. The resultant solution is then mixed with a poor solvent. Crystals thus precipitated are collected by filtration and then dried into pellets or powder. As an alternative, after their dissolution in an organic solvent, the compounds are partially cured to the stage of a prepolymer by heat treatment. The prepolymer is then mixed with a poor solvent. Crystals thus precipitated are collected by filtration and then dried into pellets or powder. Conditions for the heat treatment should follow those employed in method (1).

The organic solvents used should not react with both reactants to any substantial extent. In addition, it is desirable that they are good solvents for both reactants. Such solvents include halogenated hydrocarbons such as methylene chloride, dichloroethane and trichloroethylene, ketones such as acetone, methyl ethyl ketone, cyclohexanone and diisopropyl ketone, ethers such as tetrahydrofuran, dioxane and methyl-cellosolve, aromatic compounds such as benzene, toluene and chlorobenzene, and aprotic polar solvents such as acetonitrile, N,N-dimethylformamide, N,N-dimethylacetamide, dimethylsulfoxide, N-methyl-2-pyrrolidone and 1,3-dimethyl-2-imidazolidinone.

The aromatic amine resin composed of the mixture of the aromatic amine compounds represented by the formula (a) may be used in an amount of 5-100 parts by weight, preferably 10-80 parts by weight per 100 parts by weight of N,N'-4,4'-diphenylmethanebismaleimide represented by the formula (e).

If the amount of the aromatic amine resin is smaller than 5 parts by weight, cured products obtained from the resultant composition will be extremely brittle and will not provide any satisfactory flexural strength. Any amounts greater than 100 parts by weight will result in cured products having poor heat resistance.

The following components may be added to the thermosetting resin compositions of this invention as needed to such an extent that the objects of this invention are not impaired.

(a) Curing accelerators, for example, radical polymerization initiators such as azo compounds and organic peroxides, and ionic catalysts such as tertiary amines, quaternary ammonium salts, imidazoles, boron trifluoride and amine salts.

(b) Powdery reinforcing materials and fillers, for example, metal oxides such as aluminum oxide and magnesium oxide, metal hydroxides such as aluminum hydroxide, metal carbonates such as calcium carbonate and magnesium carbonate, diatomaceous earth, basic magnesium silicate, calcined clay, fine particulate silica, molten silica, crystalline silica, carbon black, kaolin, fine particulate mica, silica flour, graphite, asbestos, molybdenum disulfide, antimony trioxide. In addition, fibrous reinforcing materials and fillers, for example, inorganic fibers such as glass fibers, rock wool, ceramic fibers, alumina fibers and potassium titanate fibers, and organic fibers such as carbon fibers and aromatic polyamide fibers.

(c) One or more of synthetic resins may also be blended in order to improve the properties of the resin composition in end products such as coating films, bonding layers and resin moldings. Such synthetic resins may include thermosetting resins such as phenol resins, epoxy resins, melamine resins and silicone resins, polyamides, polycarbonates, polysulfones, polyether sulfones, polyether ether ketones, modified polyphenylene oxides, polyphenylene sulfides, polyether imides, and fluoroplastics.

The thermosetting resin compositions of this invention can be molded or otherwise formed for practical applications by known molding or forming methods such as compression molding, transfer molding, extrusion and injection molding.

This invention will hereinafter be described in more detail by the following Examples.

Example 1:

A reaction vessel fitted with a stirrer, a thermometer and a Dean-Stark's azeotropic distillation trap was charged with 111.6 g (1.2 moles) of aniline as an aromatic amine compound represented by the general formula (b), 66.5 g (0.4 mole) of $\alpha,\alpha'$-dimethoxy-p-xylene as an aralkyl alcohol derivative represented by the general formula (c) and 62.6 g (0.6 mole) of a 35% aqueous solution of hydrochloric acid as a catalyst. The contents were heated while feeding nitrogen gas. Water which began to be distilled out into the trap from an internal temperature of about 110 °C was taken out of the system. When the internal temperature was raised further, distillation of methanol was observed from about 130 °C. While distilling out methanol thus formed, the internal temperature was continuously raised. After reaching 170 °C, the reaction mixture was maintained at the same temperature for 3 hours. Formation of methanol ceased substantially. The reaction mixture was thereafter heated further to 190-200 °C, at which the reaction was allowed to proceed for 12

hours. The reaction system was then cooled to lower the internal temperature to 95°C. To the thus-cooled reaction mixture, 168 g of a 15% aqueous solution of caustic soda was added. They were stirred to neutralize the reaction mixture. After allowing the resultant mixture to stand, a water layer appeared as a lower layer and was separated out. Saturated saline (300 g) was added to wash the upper layer. The saline was then separated out. The resultant solution was heated and dried under a nitrogen gas stream and was then filtered under pressure to remove inorganic salts and the like. The filtrate was concentrated in a vacuum of 2-3 mmHg to recover 51.9 g of unreacted aniline. The residue was taken out, thereby obtaining 94.5 g of an aromatic amine resin of a pale yellowish brown color.

The composition of the aromatic amine resin was analyzed by high-performance liquid chromatography. It was found to have the following composition:

| Amine compound(s) of general formula (a) | n = 0 | n = 1 | n = 2 | n = 3 | n≧4 |
|---|---|---|---|---|---|
| Mole % | 28 | 16.8 | 10.5 | 7.8 | 36.9 |

Further, the amine equivalent of the resin as measured by the perchloric acid-glacial acetic acid method was 0.578 equivalent/100 g. Its softening point as measured by a ring and ball softening point measuring instrument according to JIS (Japanese Industrial Standard) K-2548 was 68°C. In addition, its average molecular weight was 960.

Results of an IR analysis of the resin are shown in FIG. 1.

Example 2:

A reaction was conducted in the manner described in Example 1 except for the use of 244.4 g (2.0 moles) of 2,4-diaminotoluene as an aromatic amine compound represented by the general formula (b) and 209 g (2.0 moles) of 35% hydrochloric acid as a catalyst, whereby 132 g of an aromatic amine resin was obtained as a reddish brown oil.

The composition of the aromatic amine resin was analyzed by high-performance liquid chromatography. It was found to have the following composition:

| Amine compound(s) of general formula (a) | n = 0 | n = 1 | n = 2 | n≧3 |
|---|---|---|---|---|
| Mole % | 44.5 | 29.7 | 14.6 | 11.2 |

The amine equivalent of the resin was 1.204. Its softening point and average molecular weight were 46°C and 550 respectively.

Example 3:

A reaction was conducted in the manner described in Example 1 except for the use of 121.1 g (1.3 moles) of aniline as an aromatic amine compound represented by the general formula (b), 138.28 g (1.0 mole) of α,α'-dihydroxy-m-xylene as an aralkyl alcohol derivative represented by the general formula (c) and 33 g (0.325 mole) of concentrated sulfuric acid as a catalyst, whereby 151 g of an aromatic amine resin of a pale yellowish brown color was obtained.

The amine equivalent of the thus-obtained aromatic amine resin was 0.496. Its softening point as measured by the ring and ball softening point measuring instrument according to JIS (Japanese Industrial Standard) K-2548 was 118°C. Its average molecular weight was 6500.

Example 4:

A reaction vessel was charged with 109 g (1.0 mole) of p-aminophenol as an aromatic amine compound represented by the general formula (b), 110.2 g (0.5 mole) of α,α'-diacetoxy-p-xylene as an aralkyl alcohol derivative represented by the general formula (c), and 6.8 g (0.05 mole) of zinc chloride and 19 g (0.1 mole) of p-toluenesulfonic acid as catalysts. They were reacted under reduced pressure by means of a water jet pump. The reaction started from about 130°C and the internal temperature arose to 170°C in 3 hours. Acetic acid generated in the course of the reaction was recovered in a deep-cooling trap. After maintaining the reaction mixture at the same temperature for 3 hours, the reaction temperature was raised

further to 200 °C, followed by aging at 200-210 °C for 1 hour before completion of the reaction. The reaction mixture was cooled down to 95 °C, at which 300 mℓ of toluene were added to dissolve the reaction mixture while stirring. After adding 20.2 g of triethylamine to the resultant mixture, 200 mℓ of water were added. The mixture was stirred and then allowed to stand. A water layer occurred as a lower layer and was separated out. An upper layer was washed once again with 200 mℓ of water and water was thereafter separated out. The resulting solution was concentrated in a vacuum to remove toluene and unreacted p-aminophenol. As a brown resin, 138 g of an aromatic amine resin was obtained.

The amine equivalent of the aromatic amine resin was 0.525. Its softening point as measured by the ring and ball softening point measuring instrument according to JIS (Japanese Industrial Standard) K-2548 was 94 °C. Its average molecular weight was 2200.

Example 5:

A reaction was conducted in the manner described in Example 1 except for the use of 745 (8.0 moles) of aniline, 664 g (4.0 moles) of α,α'-dimethoxy-p-xylene and 420 g (4.0 moles) of a 35% aqueous solution of hydrochloric acid as a catalyst, whereby 747 g of an aromatic amine resin of a pale yellowish brown color was obtained.

The composition of the aromatic amine resin was analyzed by high-performance liquid chromatography. It was found to have the following composition:

| Amine compound(s) of general formula (a) | n = 0 | n = 1 | n = 2 | n≧3 |
|---|---|---|---|---|
| Mole % | 17.0 | 14.5 | 13.2 | 55.2 |

The amine equivalent of the resin was 0.520 equivalent/100 g. Its softening point and average molecular weight were 61 °C and 2100 respectively.

Examples 6-14:

Various aromatic amine resins of this invention as shown in Table 1 were obtained by separately conducting reactions in the manner described in Example 1 except that the aromatic amine compound represented by the general formula (b), the aralkyl alcohol derivative represented by the general formula (c) and the catalyst and the reaction conditions were selected as shown in Table 1.

Table 1 Syntheses of Various Aromatic Amine Resins

| Ex. | Aromatic amine | | Aralkyl alcohol derivatives | | Catalyst | | Reaction conditions | Synthesized resin | | | |
|---|---|---|---|---|---|---|---|---|---|---|---|
| | | Amount (mole) | | Amount (mole) | | Amount (mole) | Temp./time (°C) (hr) | Structural unit | Yield (g) | M.W. | S.P.* (°C) |
| 6 | o-Toluidine | 1.2 | 4,4'-Dihydroxymethyldiphenyl ether | 0.12 | HCl | 0.4 | 140-170/5<br>170-195/12 | 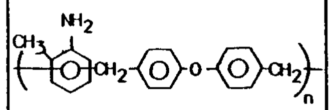 | 50.5 | 580 | Oily |
| 7 | 2,4-Xylidine | 1.2 | α,α'-Diisopropoxy-p-xylene | 0.4 | ditto | 0.6 | 130-170/5<br>170-200/8 | 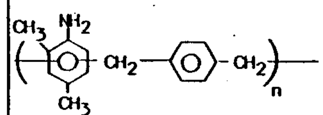 | 116 | 1350 | 75 |
| 8 | 2,4,6-Trimethylaniline | 1.2 | α,α'-Dihydroxy-2,5-dimethyl-p-xylene | 0.3 | ditto | 0.24 | 130-170/5<br>200-210/8 | 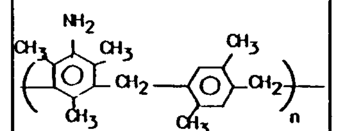 | 92 | 850 | 70 |
| 9 | p-n-Propylaniline | 1.2 | α,α'-Dimethoxy-p-xylene | 1.0 | ditto | 0.6 | 130-150/5<br>180-200/14 | 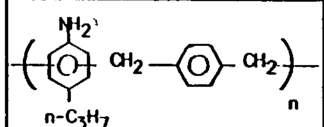 | 246 | 12000 | 142 |
| 10 | m-Isopropoxyaniline | 1.2 | ditto | 0.4 | ditto | 0.24 | 130-150/3<br>170-190/6 | 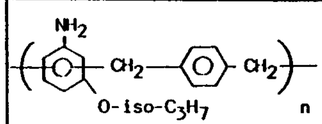 | 120 | 1200 | 56 |

* Softening point as measured by the ring and ball softening point measuring apparatus according to JIS (Japanese Industrial Standard) K-2548.

Table 1 (Cont'd)

| Ex. | Aromatic amine | | Aralkyl alcohol derivatives | | Catalyst | | Reaction conditions | Synthesized resin | | | |
|---|---|---|---|---|---|---|---|---|---|---|---|
| | | Amount (mole) | | Amount (mole) | | Amount (mole) | Temp./time (°C) (hr) | Structural unit | Yield (g) | M.W. | S.P.* (°C) |
| 11 | p-Chloro-aniline | 1.2 | α,α'-Dimethoxy-P-xylene | 0.4 | $H_3PO_4$ | 0.24 | 150-170/3 190-200/8 | 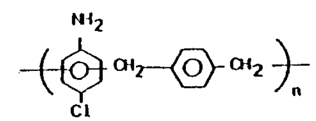 | 118 | 1150 | 78 |
| 12 | 4-Chloro-o-toluidine | 1.2 | ditto | 0.4 | HCl | 0.6 | 150-170/5 190-200/12 | 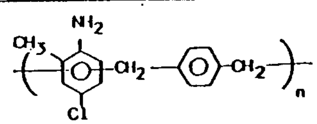 | 120 | 1100 | 75 |
| 13 | 3,5-Dichloro-4-aminophenol | 1.2 | ditto | 0.4 | ditto | 0.6 | 150-170/5 170-190/12 | 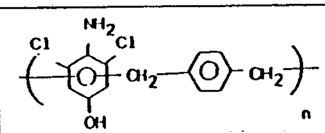 | 135 | 1250 | 75 |
| 14 | 5-tert-Butyl-o-toluidine | 1.2 | ditto | 0.4 | $CF_3SO_3H$ | 0.15 | 130-150/3 170-190/6 | 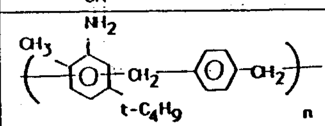 | 142 | 1520 | 87 |

* Softening point as measured by the ring and ball softening point measuring apparatus according to JIS (Japanese Industrial Standard) K-2548.

Application Example 1:

In 1400 g of dry o-dichlorobenzene, 100 g of the aromatic amine resin obtained in Example 1 were dissolved. Thereafter, 253 g of phosgene was blown at 5-10°C into the solution over 3 hours. Simultaneously, the solution was heated and was aged at 120-140°C for 2 hours. The feed of phosgene was then

stopped and replaced by a feed of nitrogen gas. The reaction system was thoroughly purged with nitrogen and was then cooled. The reaction mixture was concentrated in a vacuum to recover o-dichlorobenzene as the solvent, so that 111 g of an isocyanated resin was obtained in the form of a pale brown oil.

In 400 g of methylene chloride, 10.4 g of the isocyanated resin obtained as described above was dissolved to provide "Solution A". "Solution B", which consisted of 2.2 g of "EDTDA" (product of Ethyl Corporation), 10 g of "JEFFAMINE T-5000" (trade name; product of Texaco Chemical Company) and 2500 g of methylene chloride, was then mixed with "Solution A". The resultant mixture was cast on a glass plate. It was allowed to stand overnight, followed by post curing at 120°C for 2 hours. A good polyurea film was successfully obtained.

Application Example 2:

Forty (40) parts by weight of the aromatic amine obtained in Example 1 were mixed with 100 parts by weight of "Bismaleimide-s" (trade name; product of Daiwa Kasei Co., Ltd.). The resultant mixture was heated and kept molten at 180°C for 10 minutes to prepare a prepolymer.

The solubility of the prepolymer in various solvents was measured. Results are summarized under Experiment 1 in Table 2. In addition, the external appearance, softening point, gelling time and bulk specific gravity of the prepolymer are summarized under Experiment 1 in Table 3. For the sake of comparison, commercial "Kerimid-1050" (trade name; molding grade; product of Nippon Polyimide Co., Ltd.) was also tested similarly. Results are shown under Comparative Experiment 1 in Table 2 and Table 3 respectively.

Next, the prepolymer and "Kerimid-1050" were separately subjected to compression molding at 200°C under 40 kgf/$cm^2$ for 1 hour. The resultant moldings were thereafter post-cured at 250°C for 4 hours, thereby producing test pieces of cured resins. Mechanical strengths and thermal properties of the test pieces were measured. Results are shown respectively under Experiment 1 and Comparative Experiment 1 in Table 3.

Table 2

Solubility of Heat-Cured Prepolymer (wt.%)

Measured at 25°C

| | Experiment 1 | Comparative Experiment 1 |
|---|---|---|
| Solvent | Aromatic amine resin/bismaleimide-s | Kerimid-1050 |
| N-Methylpyrrolidone | >50 | >50 |
| N,N-Dimethylacetamide | >50 | >50 |
| N,N-Dimethylformamide | >50 | 48 |
| 1,4-Dioxane | 35 | <1 |
| Diethylene glycol dimethyl ether | <1 | <1 |
| m-Cresol | 38 | 34 |
| Methylene chloride | 28 | <1 |
| Trichloroethylene | <1 | <1 |
| Benzene | <1 | <1 |
| Toluene | <1 | <1 |
| Methoxybenzene | <1 | <1 |
| Acetone | <1 | <1 |
| Methyl ethyl ketone | <1 | <1 |

Table 3 Basic Physical Properties

| | Tested property | Testing method | Unit | Experiment 1 | Comparative Experiment 1 |
|---|---|---|---|---|---|
| | | | | Aromatic amine resin/bismaleimide-s | Kerimid-1050 |
| Prepolymer | External appearance | - | - | Yellow powder | Yellow powder |
| | Softening point | Capillary method | °C | 105 | 107 |
| | Gelling time | 200°C hot plate method | sec | 62-73 | 56-68 |
| | Bulk specific gravity | JIS K-6911 | g/cc | 0.44 | 0.42 |
| Molding (post-cured at 250°C for 4 hours) | Specific gravity | JIS K-6911 | - | 1.30 | 1.31 |
| | Flexural strength | JIS K-6711, 25°C | $kg/mm^2$ | 12.8 | 8.6 |
| | Flexural modulus | JIS K-6911, 25°C | $kg/mm^2$ | 372 | 352 |
| | Izod impact strength | JIS K-6911, un-notched | kg·cm/cm | 18 | 13 |
| | Barcol hardness | ASTM D 2583 | - | 45 | 47 |
| | Heat distortion temp. | JIS K-6911, $18.5/cm^2$ | °C | >260 | >260 |
| | Pyrolysis starting temperature | TGA method, heating rate: 10°C/min | °C | 341 | 322 |
| | Glass transition temperature | TMA penetration method | °C | >300 | >300 |
| | Expansion coefficient | JIS K-6911 | $°C^{-1}$ | $5.69x10^{-5}$ | $5.89x10^{-5}$ |
| | Water absorption rate | 25°C/24 hrs, immersed in water | % | 0.48 | 0.91 |

Example 15:

A reaction vessel fitted with a stirrer and a thermometer was charged with 111.6 g (1.2 moles) of aniline as an aromatic amine compound represented by the general formula (b) and 70.0 g (0.4 mole) of α,α'-dichloro-p-xylene as a bishalogenomethyl derivative represented by the general formula (d). The contents were heated while feeding nitrogen gas. Although evolution of heat was observed from an internal temperature of about 30 °C, the contents were continuously heated and maintained at 85-100 °C for 3 hours (first-stage reaction). They were thereafter heated and reacted at 190-200 °C for 20 hours (second-stage

reaction). The reaction system was then cooled to lower the internal temperature to 95 ° C. To the cooled reaction mixture, 230 g of a 15% aqueous solution of caustic soda were added and stirred to neutralize the reaction mixture. After allowing the mixture to stand, a water layer appeared as a lower layer and was separated out. Saturated saline (300 g) was added to the upper layer to wash it. The resultant solution was heated and dried under a nitrogen gas stream and was then filtered under pressure to remove inorganic salts and the like. The filtrate was concentrated in a vacuum of 2-3 mmHg to recover 48.5 g of unreacted aniline. The residue was taken out, thereby obtaining 100 g of an aromatic amine resin of a pale yellowish brown color.

The composition of the aromatic amine resin was analyzed by high-performance liquid chromatography. It was found to have the following composition:

| Amine compound(s) of general formula (a) | n = 0 | n = 1 | n = 2 | n = 3 | n≧4 |
|---|---|---|---|---|---|
| Mole % | 27.8 | 19.2 | 14.0 | 11.8 | 27.2 |

Further, the amine equivalent of the resin as measured by the perchloric acid-glacial acetic acid method was 0.65 equivalent/100 g. Its softening point as measured by a ring and ball softening point measuring instrument according to JIS (Japanese Industrial Standard) K-2548 was 64 ° C. In addition, its average molecular weight was 880.

Example 16:

The procedure of Example 15 was repeated except that 61.1 g (0.5 mole) of 2,4-diaminotoluene and 29.2 g (0.1 mole) of α,α'-dibromo-2,5-dimethyl-p-xylene were used as an aromatic amine compound represented by the general formula (b) and a bishalogenomethyl derivative represented by the general formula (d) respectively and the reaction time of the second stage was changed to 30 hours, whereby 32.5 g of an aromatic amine resin was obtained.

The composition of the aromatic amine resin thus obtained was analyzed by high-performance liquid chromatography. It was found to have the following composition:

| Amine compound(s) of general formula (a) | n = 0 | n = 1 | n = 2 | n≧3 |
|---|---|---|---|---|
| Mole % | 41.8 | 21.2 | 13.1 | 23.9 |

The amine equivalent of the resin was 1.212. Its softening point and average molecular weight were 46 ° C and 520 respectively.

Example 17:

The procedure of Example 15 was repeated except for the use of 53.6 g (0.5 mole) of mixed toluidine, which consisted of 19% of the o-isomer, 53% of the m-isomer and 28% of the p-isomer, and 106.9 g (0.4 mole) of 4,4'-bis(chloromethyl)diphenyl ether as an aromatic amine compound represented by the general formula (b) and a bishalogenomethyl derivative represented by the general formula (d) respectively, whereby 116 g of an aromatic amine resin was obtained.

The amine equivalent of the aromatic amine resin obtained as described above was 0.35. Its softening point as measured by the ring and ball softening point measuring instrument according to JIS (Japanese Industrial Standard) K-2548 was 136 ° C. Its average molecular weight was 13200.

Example 18:

A reaction vessel similar to that employed in Example 15 was charged with 93.1 g (1 mole) of aniline as an aromatic amine compound represented by the general formula (b) and 17.5 g (0.1 mole) of α,α'-dichloro-m-xylene as a bishalogenomethyl derivative represented by the general formula (d). The contents were heated while feeding nitrogen gas. Although evolution of heat was observed from an internal temperature of about 30 ° C, the contents were continuously heated and maintained at 70-80 ° C for 5 hours (first-stage reaction). Next, 31.3 g (0.3 mole) of a 35% aqueous solution of hydrochloric acid was charged into the reaction mixture, and the resultant mixture was heated. Water formed in the course of the heating was

distilled out of the system. The reactants were thereafter reacted at 180-190°C for 16 hours (second-stage reaction). Cooling, stirring and neutralization, separation and removal, washing and separation, heating and drying, pressure filtration and vacuum concentration were then carried out as described in Example 15, thereby obtaining 25 g of an aromatic amine resin as a pale brown oil.

The composition of the aromatic amine resin obtained as described above was analyzed by high-performance liquid chromatography. It was found to have the following composition:

| Amine compound(s) of general formula (a) | n=0 | n=1 | n=2 | n=3 |
|---|---|---|---|---|
| Mole % | 79.0 | 17.7 | 2.7 | 0.6 |

Further, the amine equivalent of the resin was 0.674, and its average molecular weight was 330.

Example 19:

The procedure of Example 18 was repeated except for the use of 17.5 g (0.1 mole) of α,α'-dichloro-p-xylene as a bishalogenomethyl derivative represented by the general formula (d) and the use of 19.0 g (0.1 mole) of p-toluenesulfonic acid as a reaction accelerator before the second-stage reaction, thereby obtaining 25.8 g of an aromatic amine resin as a pale brown oil.

The amine equivalent of the aromatic amine resin obtained was 0.671. Its composition was substantially the same as the resin obtained in Example 18.

Examples 20-23:

A stainless reaction vessel fitted with a stirrer, a reflux condenser and a nitrogen inlet tube was charged with N,N'-4,4'-diphenylmethanebismaleimide and the aromatic amine resin which was obtained in Example 1, in amounts given respectively in terms of parts by weight in Table 4. The contents were heated and kept molten at 180°C for 20 minutes, followed by defoaming at 150°C under reduced pressure (10-15 mmHg) for 30 minutes. The melt was then cooled to room temperature. In this manner, resin compositions were separately obtained in a form which solidified in a brown transparent glassy state.

While separately heating and melting the compositions, they were individually filled into molds heated at 180°C and were then maintained under 50 kgf/cm$^2$ at 200°C for 30 minutes to perform compression molding. Resultant moldings were taken out of the respective molds and then post-cured for 4 hours in an oven of 250°C, thereby obtaining test pieces of cured resins whose length, width and thickness were 127 mm, 12.7 mm and 6.4 mm respectively.

The heat distortion temperatures of those test pieces were measured in accordance with ASTM D-648, while their bending tests were conducted in accordance with ASTM D-790. In addition, their pyrolysis starting temperatures were also measured at a heating rate of 10°C/min in air. Results are summarized in Table 4.

Examples 24-36 & Comparative Examples 1-2:

The procedure of Examples 20-23 was repeated using 100 parts by weight of N,N'-4,4'-diphenylmethanebismaleimide and the aromatic amine resins, which are given in Table 4, in amounts indicated in terms of parts by weight in Table 4. Results are also shown in Table 4.

Comparative Example 3:

The procedure of Examples 20-23 was repeated except for the use of N,N'-4,4'-diphenylmethanebismaleimide and 4,4'-diaminodiphenylmethane in their respective amounts shown in terms of parts by weight in Table 4. Results are also given in Table 4.

Comparative Example 4:

Moldings were produced in the same manner as in Examples 20-23 by using "Kerimid-1050" (trade name for a polyaminobismaleimide resin; product of Japan Polyimide Co., Ltd.) instead of the resin compositions of this invention. Various physical properties were measured. Results are also given in Table 4.

From the results shown in Table 4, the thermosetting resin compositions of this invention are high in both flexural strength and flexural modulus and also have excellent heat resistance as demonstrated by their heat distortion temperatures as high as 290 °C and up and their pyrolysis starting temperatures as high as 340 °C and up.

Table 4 Examples 20-36 & Comparative Examples 1-4

| | Bismaleimide (wt. parts) | Aromatic amine resin (wt. parts) | Flexural strength ($kgf/mm^2$) | Flexural modulus ($kgf/mm^2$) | Heat distortion (°C) | Pyrolysis starting temp. (°C) |
|---|---|---|---|---|---|---|
| Ex.20 | N,N'-4,4'-Diphenylmethanebismaleimide (100) | Ex. 1 (10) | 10.2 | 351 | >300 | 371 |
| Ex.21 | ditto (100) | ditto (30) | 12.0 | 365 | >300 | 369 |
| Ex.22 | ditto (100) | ditto (50) | 12.8 | 372 | >300 | 362 |
| Ex.23 | ditto (100) | ditto (80) | 11.5 | 370 | 297 | 357 |
| Ex.24 | ditto (100) | Ex. 5 (50) | 11.9 | 389 | >300 | 360 |
| Ex.25 | ditto (100) | Ex. 2 (50) | 11.9 | 375 | 294 | 354 |
| Ex.26 | ditto (100) | Ex. 3 (50) | 12.6 | 370 | >300 | 361 |

Table 4 (Cont'd)

| | Bismaleimide (wt. parts) | Aromatic amine resin (wt. parts) | Flexural strength (kgf /mm$^2$) | Flexural modulus (kgf /mm$^2$) | Heat distortion (°C) | Pyrolysis starting temp. (°C) |
|---|---|---|---|---|---|---|
| Ex.27 | N,N'-4,4'-Diphenylmethanebismaleimide (100) | Ex. 4 (50) | 11.3 | 369 | >300 | 356 |
| Ex.28 | ditto (100) | Ex. 6 (50) | 13.1 | 389 | 290 | 350 |
| Ex.29 | ditto (100) | Ex. 7 (50) | 11.7 | 387 | 294 | 342 |
| Ex.30 | ditto (100) | Ex. 8 (10) | 12.0 | 375 | 297 | 349 |
| Ex.31 | ditto (100) | Ex. 9 (50) | 12.0 | 380 | >300 | 351 |
| Ex.32 | ditto (100) | Ex. 10 (50) | 12.4 | 370 | >300 | 350 |
| Ex.33 | ditto (100) | Ex. 11 (50) | 11.9 | 383 | >300 | 351 |

Table 4 (Cont'd)

| | Bismaleimide (wt. parts) | Aromatic amine resin (wt. parts) | Flexural strength (kgf/mm²) | Flexural modulus (kgf/mm²) | Heat distortion (°C) | Pyrolysis starting temp.(°C) |
|---|---|---|---|---|---|---|
| Ex.34 | N,N'-4,4'-Diphenylmethanebismaleimide (100) | Ex. 12 (50) | 11.7 | 383 | >300 | 352 |
| Ex.35 | ditto (100) | Ex. 13 (50) | 12.0 | 371 | >300 | 353 |
| Ex.36 | ditto (100) | Ex. 14 (50) | 12.2 | 376 | >300 | 341 |
| Comp. Ex.1 | ditto (100) | - (0) | Molding was infeasible. (Cracks were formed.) | | | 418 |
| Comp. Ex.2 | ditto (100) | Ex. 1 (150) | 8.9 | 349 | 280 | 331 |
| Comp. Ex.3 | ditto (100) | 4,4'-diamino-diphenylmethane (30) | 8.6 | 352 | 276 | 330 |
| Comp. Ex.4 | Kerimid-1050 | | 8.6 | 352 | 285 | 333 |

As described above, the aromatic amine resins of this invention are composed of primary amines and hence facilitate isocyanation, maleimidation and epoxidation. Accordingly, they are also useful as starting materials for polyamides and the like.

When the aromatic amine resins of this invention are used as curing agents or starting materials for other resins, the resulting resins have excellent mechanical strength, dimensional stability, heat resistance,

and stability to light and oxygen in air, and their curing speeds are also high.

The aromatic amine resins of this invention can readily provide prepolymers because they are soluble in solvents of low melting point.

Since the aromatic amine resins of this invention have such advantageous effects, they can be used in a wide variety of fields, for example, as curing agents, raw materials for cured resins, chelate resins, ion-exchange resins, molding materials, insulating paints, bonding agents, rubber modifiers, additives to various resins, deacidification agents, and raw materials for polyimides, polyamides and polyamideimides.

Further, the aromatic amine resins of this invention can be produced from economical starting materials by a simple operation, and can be obtained by a process which is practically free of pollution and byproducts. It is also possible to produce aromatic amine resins in various forms, ranging from those in a liquid form at room temperature to those in a resinous form having a high softening point, in accordance with the end use. In the second process of this invention in particular, there are such advantages that catalyst is not absolutely required for the reaction, the aromatic amine resins can be produced economically, and less side reactions take place.

In addition, the thermosetting resins according to this invention have superb mechanical strength and heat resistance. They are hence expected to find wide-spread utility in fields in which high heat resistance is required in recent years, for example, the electrical and electronic field, the mechanical field such as aircrafts and vehicles, and the like field. They can therefore be used advantageously in industry.

## Claims

**1.** An aromatic amine resin formed of a mixture of aromatic amines of the general formula (a):

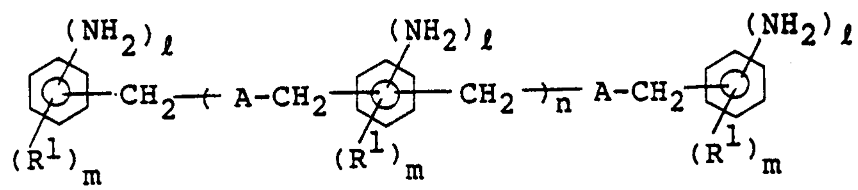

(a)

in which A is phenylene, alkyl-substituted phenylene, diphenylene, diphenyl ether or naphthylene, $R^1$ is halogen or hydroxyl, $C_{1-4}$ alkoxy or $C_{1-5}$ alkyl, $\ell$ is 1 or 2, m is O or an integer from 1 to 3 and when m is 2 or 3, then the $R^1$s may be the same or different and two $R^1$s may together form a 5- or 6-membered alicyclic moiety which may optionally contain one or more side chains, and n has a value from 0 to 300.

**2.** An aromatic amine resin according to claim 1, wherein m is 0 and $\ell$ is 1.

**3.** An aromatic amine resin according to claim 1 or claim 2, wherein n has a value from 0 to 4.

**4.** An aromatic amine resin according to any one of claims 1 to 3, wherein A is a phenylene group.

**5.** A process for the production of an aromatic amine resin according to claim 1, which comprises reacting together in the presence of an acid catalyst an aromatic amine of the general formula (b):

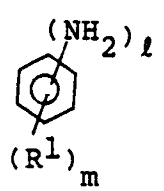

(b)

in which $R^1$, $\ell$ and m have the meanings specified in claim 1, and an aralkyl alcohol derivative of the general formula (c):

$R^2OCH_2$-A-$CH_2OR^2$ (c)

in which A has the meaning specified in claim 1 and $R^2$ is hydrogen, acyl or $C_{1-4}$ alkyl, and wherein:
(i) the compounds (b) and (c) are in a molar ratio of 1 to 15;
(ii) the amount of acid catalyst is 10 to 100 mole % of the aromatic amine;
(iii) the temperature is 130 to 240 °C; and
(iv) the reaction time is 10 to 40 hours.

**6.** A process for the production of an aromatic amine resin according to claim 1, which comprises reacting together an aromatic amine of the general formula (b):

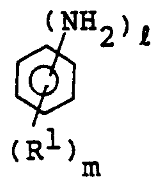

(b)

in which $R^1$, $\ell$ and m have the meanings specified in claim 1, and a bishalomethyl derivative of the general formula (d):

$X\text{-}CH_2\text{-}A\text{-}CH_2\text{-}X$ (d)

in which A has the meaning specified in claim 1 and X is halogen, and wherein:
(i) the compounds (b) and (c) are in a molar ratio of 1 to 15;
(ii) the temperature is O to 240 °C, and
(iii) the reaction time is 6 to 50 hours.

**7.** A process according to claim 6, wherein the aromatic amine (b) and the bishalomethyl derivative (d) are reacted together in the presence of an acid catalyst in an amount of 10 to 100 mole % of the aromatic amine.

**8.** A process for the production of an aromatic amine resin according to claim 1, which comprises reacting together in a two stage process an aromatic amine of the following formula (b):

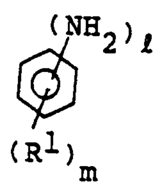

(b)

in which $R^1$, $\ell$ and m have the meanings specified in claim 1, and a bishalomethyl derivative of the general formula (d):

$X\text{-}CH_2\text{-}A\text{-}CH_2\text{-}X$ (d)

in which A has the meaning specified in claim 1 and X is halogen, and wherein:
(i) in the first stage, the temperature is 0 to 130 °C and the reaction time is 1 to 10 hours, and
(ii) in the second stage, the temperature is 130 to 240 °C and the reaction time is 5 to 40 hours.

**9.** A thermosetting resin composition comprising 100 parts by weight of N,N'-4,4'-diphenylmethanebis-maleimide of the formula (e):

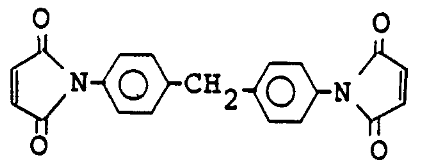

(e)

and 5 to 100 parts by weight of a resin according to any one of claims 1 to 4.

## Patentansprüche

1. Ein aromatisches Aminharz, das aus einer Mischung von aromatischen Aminen der allgemeinen Formel (a) gebildet ist:

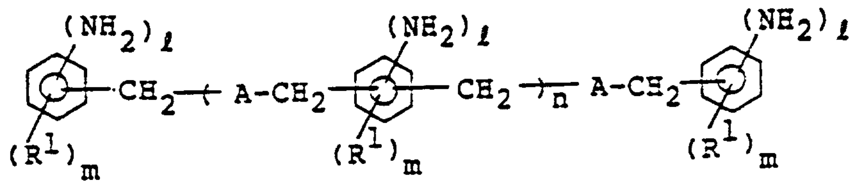

(a)

wobei A Phenylen, alkyl-substituiertes Phenylen, Diphenylen, Diphenyläther oder Naphthylen ist, $R^1$ Halogen oder Hydroxyl, $C_{1-4}$-Alkoxy oder $C_{1-5}$-Alkyl ist, $\ell$ 1 oder 2 ist, m 0 oder eine ganze Zahl von 1 bis 3 ist und, wenn m 2 oder 3 ist, dann können die $R^1$ gleich oder unterschiedlich sein und zwei $R^1$ können zusammen einen 5- oder 6-gliedrigen alicyclischen Anteil bilden, der wahlweise eine oder mehrere Seitenketten enthalten kann; und n einen Wert von 0 bis 300 aufweist.

2. Ein aromatisches Aminharz nach Anspruch 1, bei dem m 0 ist und $\ell$ = 1 ist.

3. Ein aromatisches Aminharz nach Anspruch 1 oder Anspruch 2, bei dem n einen Wert von 0 bis 4 aufweist.

4. Ein aromatisches Aminharz nach einem der Ansprüche 1 bis 3, bei dem A eine Phenylengruppe ist.

5. Ein Verfahren für die Herstellung eines aromatischen Aminharzes nach Anspruch 1, das umfaßt, daß ein aromatisches Amin der allgemeinen Formel (b):

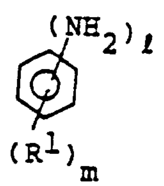

(b)

in der $R^1$, $\ell$ und m die gleichen Bedeutungen besitzen, wie sie in Anspruch 1 angegeben worden sind, und ein Aralkylalkoholderivat der allgemeinen Formel (c):

$R^2OCH_2$-A-$CH_2OR^2$, (c)

worin A die gleiche Bedeutung besitzt, wie sie in Anspruch 1 angegeben worden ist, und $R^2$ Wasserstoff, Acyl oder $C_{1-4}$-Alkyl ist, zusammen in Anwesenheit eines sauren Katalysators umgesetzt werden, und wobei:
(i) die Verbindungen (b) und (c) in einem molaren Verhältnis von 1 bis 15 stehen;
(ii) die Menge des sauren Katalysators 10 bis 100 Mol-% von dem aromatischen Amin ist;
(iii) die Temperatur 130 bis 240° C beträgt und
(iv) die Reaktionszeit 10 bis 40 Stunden ist.

6. Ein Verfahren für die Herstellung eines aromatischen Aminharzes nach Anspruch 1, das umfaßt, daß ein aromatisches Amin der allgemeinen Formel (b):

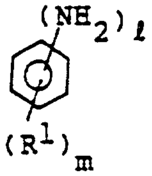

(b)

bei dem $R^1$, $\ell$ und m die gleichen Bedeutungen besitzen, wie sie in Anspruch 1 angegeben worden sind, und ein Bishalogenmethylderivat der allgemeinen Formel (d):

$X\text{-}CH_2\text{-}A\text{-}CH_2\text{-}X$, (d)

in der A die gleiche Bedeutung besitzt, wie sie in Anspruch 1 angegeben worden ist, und X Halogen ist, zusammen umgesetzt werden, und wobei:

(i) die Verbindungen (b) und (c) in einem molaren Verhältnis von 1 zu 15 stehen;
(ii) die Temperatur 0 bis 240 ° C beträgt und
(iii) die Reaktionszeit 6 bis 50 Stunden ist.

7. Ein Verfahren nach Anspruch 6, bei dem das aromatische Amin (b) und das Bishalogenmethylderivat (d) in Anwesenheit eines sauren Katalysators in einer Menge von 10 bis 100 Mol-% des aromatischen Amins zusammen umgesetzt werden.

8. Ein Verfahren für die Herstellung eines aromatischen Aminharzes nach Anspruch 1, das umfaßt, daß in einem zweistufigen Verfahren ein aromatisches Amin der folgenden Formel (b):

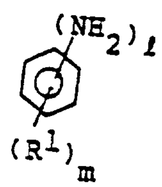

(b)

in der $R^1$, $\ell$ und m die gleichen Bedeutungen besitzen, wie sie in Anspruch 1 angegeben worden sind, und ein Bishalogenmethylderivat der allgemeinen Formel (d):

$X\text{-}CH_2\text{-}A\text{-}CH_2\text{-}X$, (d)

in der A die gleiche Bedeutung besitzt, wie sie in Anspruch 1 angegeben worden ist, und X Halogen ist, zusammen umgesetzt werden, und wobei:

(i) in der ersten Stufe die Temperatur 0 bis 130 ° C beträgt und die Reaktionszeit 1 bis 10 Stunden ist und
(ii) in der zweiten Stufe die Temperatur 130 bis 240 ° C ist und die Reaktionszeit 5 bis 40 Stunden beträgt.

9. Eine wärmehärtende Harzzusammensetzung, die 100 Gewichtsteile N,N'-4,4'-Diphenylmethanbismaleimid der Formel (e):

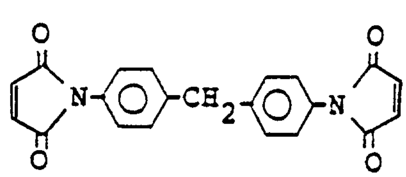

(e)

und 5 bis 100 Gewichtsteile eines Harzes gemäß einem der Ansprüche 1 bis 4 umfaßt.

**Revendications**

**1.** Une amino-résine aromatique formée d'un mélange d'amines aromatiques de formule générale (a) :

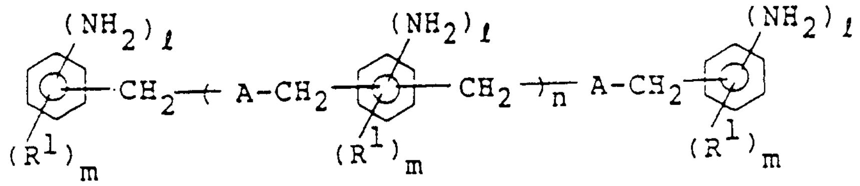

(a)

dans laquelle A est un phénylène, un phénylène alkyl-substitué, un diphénylène, un éther diphénylique ou un naphtylène, $R^1$ est un halogène ou un hydroxyle, un alcoxy en $C_{1-4}$ ou un alkyle en $C_{1-5}$, $\ell$ est 1 ou 2, m est 0 ou un entier de 1 à 3 et, lorsque m est 2 ou 3, les $R^1$ peuvent être identiques ou différents et deux $R^1$ peuvent former ensemble un fragment alicyclique à 5 ou 6 chaînons, qui peut facultativement contenir une ou plusieurs chaînes latérales, et n a une valeur de 0 à 300.

**2.** Une amino-résine aromatique selon la revendication 1, dans laquelle m est 0 et $\ell$ est 1.

**3.** Une amino-résine aromatique selon la revendication 1 ou la revendication 2, dans laquelle n a une valeur de 0 à 4.

**4.** Une amino-résine aromatique selon l'une quelconque des revendications 1 à 3, dans laquelle A est un groupe phénylène.

**5.** Un procédé pour la production d'une amino-résine aromatique selon la revendication 1, qui comprend la réaction mutuelle, en présence d'un catalyseur acide, d'une amine aromatique de formule générale (b) :

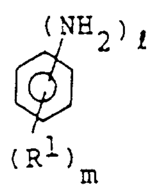

(b)

dans laquelle $R^1$, $\ell$ et m ont les significations indiquées dans la revendication 1, et d'un dérivé d'alcool aralkylique de formule générale (c) :

$R^2OCH_2\text{-}A\text{-}CH_2OR^2$ (c)

dans laquelle A a la signification indiquée dans la revendication 1 et $R^2$ est un hydrogène, un acyle ou un alkyle en $C_{1-4}$, et dans lequel

(i) les composés (b) et (c) sont dans un rapport molaire de 1 à 15 ;
(ii) la proportion du catalyseur acide est de 10 à 100 % molaires de l'amine aromatique ;
(iii) la température est de 130 à 240 °C ; et
(iv) la durée de réaction est de 10 à 40 heures.

**6.** Un procédé pour la production d'une amino-résine aromatique selon la revendication 1, qui comprend la réaction mutuelle d'une amine aromatique de formule générale (b) :

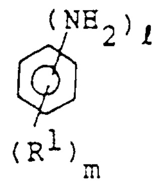

(b)

dans laquelle $R^1$, $\ell$ et m ont les significations indiquées dans la revendication 1, et d'un dérivé bishalogénométhylique de formule générale (d) :

$X\text{-}CH_2\text{-}A\text{-}CH_2\text{-}X$ (d)

dans laquelle A a la signification indiquée dans la revendication 1 et X est un halogène, et dans lequel :

(i) les composés (b) et (c) sont dans un rapport molaire de 1 à 15 ;
(ii) la température est de 0 à 240 °C ; et
(iii) la durée de réaction est de 6 à 50 heures.

**7.** Un procédé selon la revendication 6, dans lequel l'amine aromatique (b) et le dérivé bishalogénométhylique (d) sont mis à réagir ensemble en présence d'un catalyseur acide en une proportion de 10 à 100 % molaires de l'amine aromatique.

**8.** Un procédé pour la production d'une amino-résine aromatique selon la revendication 1, qui comprend la réaction mutuelle, dans une opération en deux étapes, d'une amine aromatique de formule (b) suivante :

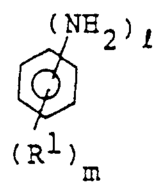

(b)

dans laquelle $R^1$, $\ell$ et m ont les significations indiquées dans la revendication 1, et d'un dérivé bishalogénométhylique de formule générale (d) :

$X\text{-}CH_2\text{-}A\text{-}CH_2\text{-}X$ (d)

dans laquelle A a la signification indiquée dans la revendication 1 et X est un halogène, et dans lequel :

(i) dans la première étape, la température est de 0 à 130 °C et la durée de réaction est de 1 à 10 heures et
(ii) dans la seconde étape, la température est de 130 à 240 °C et la durée de réaction est de 5 à 40 heures.

**9.** Une composition de résine thermodurcissable comprenant 100 parties en poids de N,N'-4,4'-diphénylméthanebismaléimide de formule (e) :

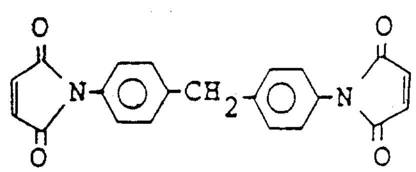

(e)

et 5 à 100 parties en poids d'une résine selon l'une quelconque des revendications 1 à 4.

FIG.1

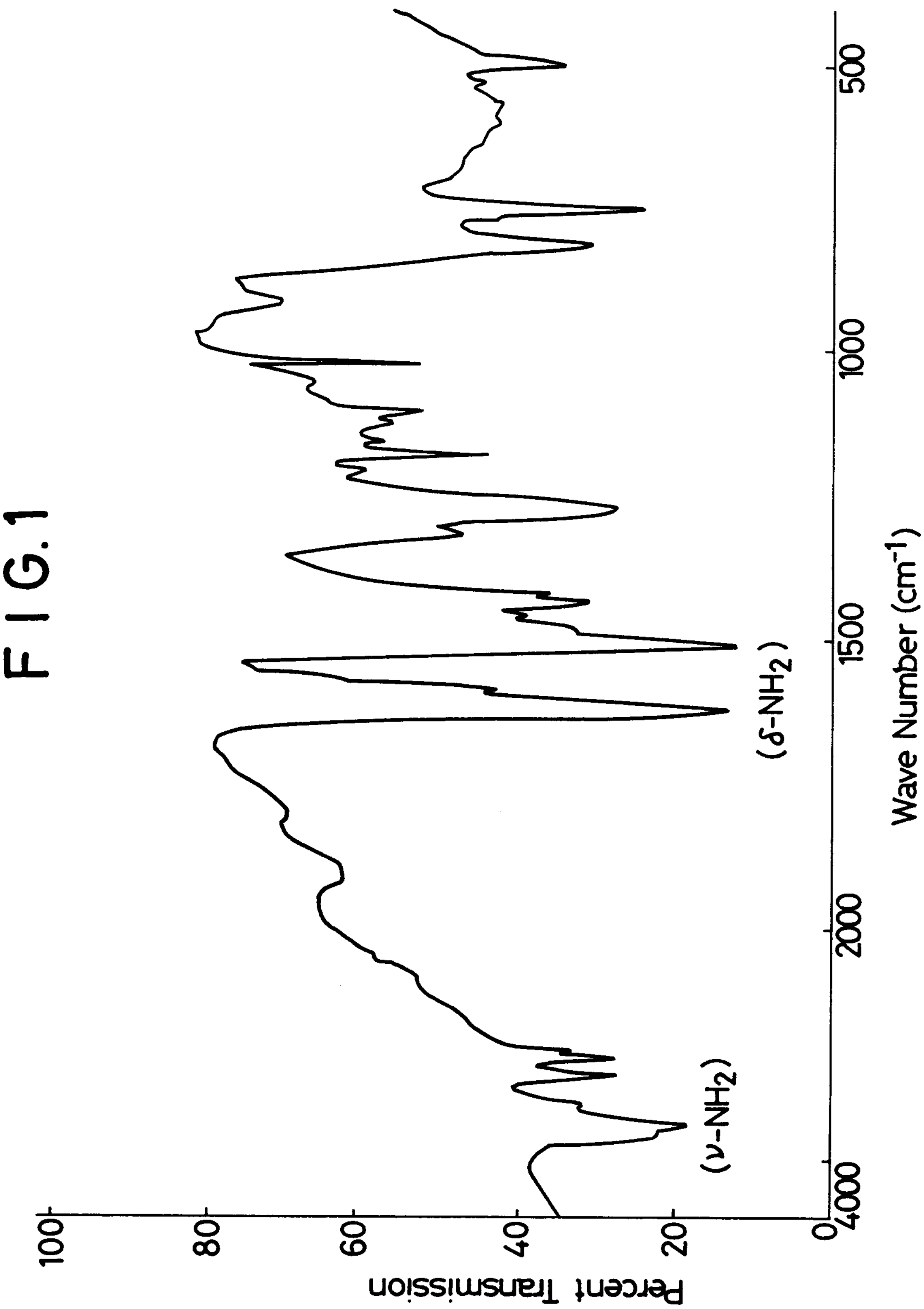
Percent Transmission
0
20
40
60
80
100
4000
2000
1500
1000
500
Wave Number ($cm^{-1}$)
($\nu$-$NH_2$)
($\delta$-$NH_2$)